Europäisches Patentamt

European Patent Office

Office européen des brevets

(19)

(11) **EP 0 849 387 A1**

(12) **EUROPEAN PATENT APPLICATION**

(43) Date of publication:
24.06.1998 Bulletin 1998/26

(51) Int. Cl.$^6$: **D04H 1/54**

(21) Application number: 96120245.4

(22) Date of filing: 17.12.1996

(84) Designated Contracting States:
AT BE CH DE DK ES FI FR GB GR IE IT LI LU NL
PT SE

(71) Applicant:
THE PROCTER & GAMBLE COMPANY
Cincinnati, Ohio 45202 (US)

(72) Inventors:
• Pollard, Ricky Alan
Moscow, Ohio 45153 (US)

• Berg, Charles John, Jr.
Wyoming, Ohio 45215 (US)
• Moore, Katherine L.H.
61462 Königstein (DE)
• Malmbak, Marianne
65510 Idstein-Eschenhahn (DE)

(74) Representative:
Canonici, Jean-Jacques et al
Procter & Gamble European Service GmbH,
Sulzbacher Strasse 40-50
65824 Schwalbach am Taunus (DE)

(54) **Disposable fibrous cleaning article**

(57) A disposable fibrous cleaning article (10) comprising a primary cleaning portion (11) disposed adjacent to at least a part of perimeter (P) of said cleaning article (10), having a primary cleaning thickness ($T_p$) ranging from 2 millimetres to 20 millimetres, and at least one secondary cleaning portion (12), characterised in that said at least one secondary cleaning portion (12) is disposed adjacent to a continuous part of said perimeter (P) of said cleaning article (10), said cleaning portion (12) having a secondary cleaning portion width ($W_s$) greater than 1 millimetre and a secondary cleaning portion thickness ($T_s$), less than said primary cleaning thickness ($T_p$), ranging from 0.01 millimetres to 3 millimetres. The at least one secondary cleaning portion (12) has a flexural rigidity value ($R_s$) of about at least 4.5E-7 newtons - metres squared, which is typically at least 100 percent greater than the flexural rigidity ($R_p$) value of the primary cleaning portion (11).

Fig. 1

EP 0 849 387 A1

## Description

### Field of the invention

The invention pertains to a disposable fibrous cleaning article having a specific portion, which functions as a secondary cleaning portion. When usage is directed to this portion of the cleaning article, a superior precision cleaning response ensues. The present invention has particular application to cleaning articles which can be used for cleaning areas of the human person, for example, skin care pads for cosmetic and therapeutic purposes, dry wipes, wet wipes and moist toilet tissue; for cleaning other surfaces, for example, kitchen and bathroom surfaces; and for surfaces which require cleaning in industry, for example, machinery or vehicle surfaces. For simplicity, the ensuing description focuses on cleaning articles for the human skin, especially facial cleansing articles, but what is said must be understood in light of the foregoing comments about the wider applicability of the present invention.

### Background of the invention

Disposable cleaning articles are well known in the art and are available both in the dry and impregnated state. Such articles typically comprise a substrate of non-woven synthetic or cellulosic fibres, which is either dry, moistened or wetted by a suitable liquid-cleansing solution. Cleaning articles with a dry-based substrate tend to be rubbed against the epidermis, generally in a circular motion, to remove and absorb dirt, grime, dead skin cells, keratin and other debris. Such articles are frequently used as carriers for cleansing products such as oils, cleansing creams, astringent solutions and cleansing lotions, which are poured directly onto the articles by the user immediately prior to application. Impregnated cleaning articles are articles saturated in skin cleansing solution for use as wipes and do not generally require as strenuous a rubbing action for efficient removal of skin debris. Both types of cleaning articles are designed to have a high tensile strength, good cleaning performance and softness. Nevertheless, a need exists for a cleaning article that exhibits an improvement in cleaning power, that cleans all areas and contours of the face especially around the more delicate regions such as the eyes, and that is soft and non-abrasive. It has thus surprisingly been found that a cleaning article having a primary cleaning portion and at least one secondary cleaning portion lying adjacent to the perimeter of the cleaning article is decidedly successful in providing benefits that are instinctively exploited by the user for a superior cleaning response. It has further been found that the above benefits are particularly enhanced with the selection of specific parameters for the cleaning articles described herein.

US 5,062,418 teaches a soft, bulky, light weight fabric with good absorbency that is suitable for use as a wound dressing or as a cleansing wipe. The four-ply folded non-woven has a bulk ranging from 1.27 millimetres to 3.05 millimetres. When subjected to a napping process, the bulk increases substantially and ranges from 2.79 millimetres to 5.33 millimetres. The patent does not disclose defined portions having different dimensional and material properties.

A line of stitching running partially around the periphery of a face washing puff of cotton pile fabric, as disclosed in US 4,932,095, could be likened to a textured portion. Nevertheless, the stitching is not designed to act as a cleaning portion and would automatically give rise to severe irritation especially around the eye regions if used for such ends.

As a result of highlighting the above prior art attempts, the benefits of the present invention are clearly evident and range from a cleaning article comprising cleaning portions with different dimensional and material properties that guarantee superior cleaning performance; to a cleaning article with a specific portion, which is preferably textured, for precision cleaning; to an effective user/product interaction to ensure a more user-friendly product; to a cleaning article that is non-abrasive and causes no irritation especially to the sensitive regions of the face; and to a cleaning article that leads to a high level of user satisfaction and confidence.

### Summary of the invention

A disposable fibrous cleaning article comprising a primary cleaning portion disposed adjacent to at least a part of perimeter of the cleaning article is disclosed. The primary cleaning portion has a primary cleaning thickness ranging from 2 millimetres to 20 millimetres, and at least one secondary cleaning portion, characterised in that at least one secondary cleaning portion is disposed adjacent to a continuous part of the perimeter of the cleaning article, the cleaning portion having a secondary cleaning portion width greater than 1 millimetre and a secondary cleaning portion thickness, less than the primary cleaning portion thickness, ranging from 0.01 millimetres to 3 millimetres. The secondary cleaning portion of the disposable fibrous cleaning article has a flexural rigidity value of about at least 4.5E-7 newtons - metres squared and this value is typically at least 100 percent greater than the flexural rigidity value for the primary cleaning portion.

In a preferred embodiment of the invention, the disposable fibrous cleaning article is directed to facial cleansing and in a more preferred embodiment of the invention, the secondary cleaning portion is applied to the regions around the eyes.

### Brief description of the drawings

It is believed that the invention will be better understood from the foregoing description in conjunction with the accompanying drawings in which:

Figure 1 is a top plan view of a disposable fibrous cleaning article showing a secondary cleaning portion;

Figure 2 is a side elevational view of the disposable fibrous cleaning article as drawn in Figure 1;

Figures 3a, 3b and 3c are top plan views of different embodiments of the disposable fibrous cleaning article according to the teachings of the present invention; and

Figure 4 is a top plan view of the disposable fibrous cleaning article showing a possible arrangement for two secondary cleaning portions.

## Detailed description of the invention

As used herein, the term "disposable" describes absorbent articles that are not intended to be laundered or otherwise restored or reused as an absorbent article (i.e., they are intended to be discarded after a single use and, preferably, to be recycled, composted or otherwise disposed of in an environmentally compatible manner). As used herein, the term "article" refers to a conventional hand-held implement that contains fibres made of cellulosic and/or synthetic material. As intended herein, the term "adjacent" implies contact with.

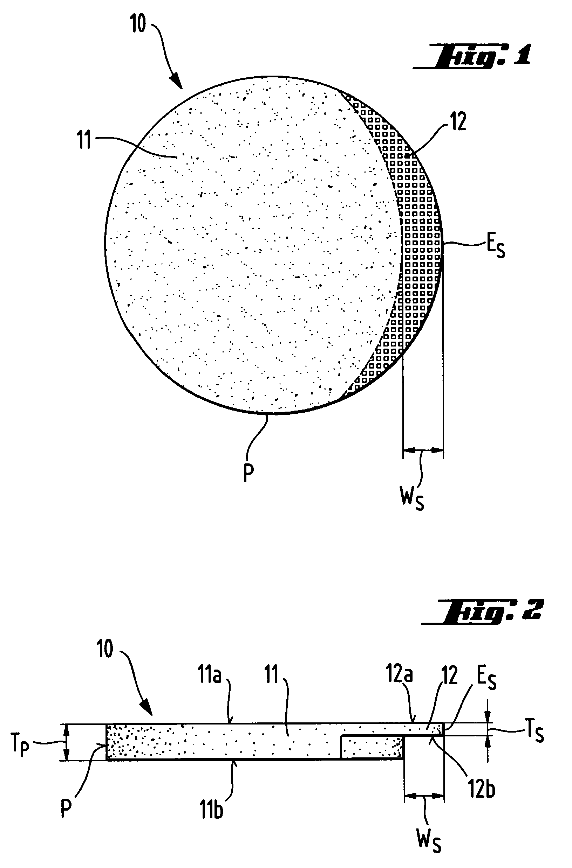

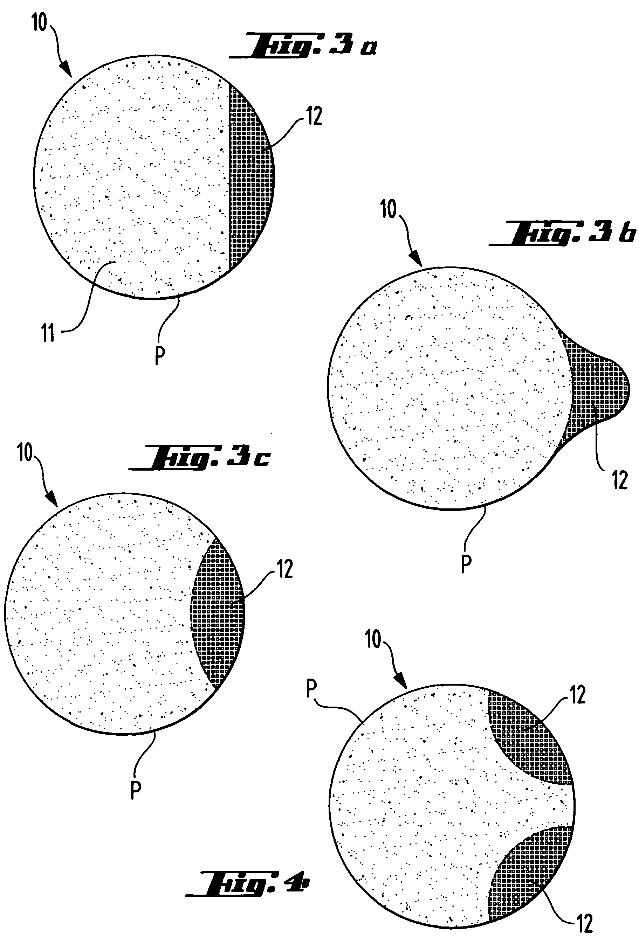

The disposable fibrous cleaning article $10$ of the present invention comprises a primary cleaning portion $11$ and at least one secondary cleaning portion $12$. In Figure 1, the cleaning article $10$ comprises two distinct portions: a primary cleaning portion $11$ and a secondary cleaning portion $12$. The cleaning article $10$ has preferably an arcuate shape, although it may comprise a variety of shapes. The examples include, but are not limited to, semi-circles, ellipsoids, polygons etc., and any combinations thereof. Regardless of the shape, the cleaning article $10$ has a perimeter $P$. In more detail, the primary cleaning portion $11$ is disposed adjacent to at least a part of the perimeter $P$ of the cleaning article $10$. The secondary cleaning portion $12$ is disposed adjacent to a continuous part of the perimeter $P$ of the cleaning article $10$. Figures 3a, 3b and 3c show various embodiments of the cleaning articles $10$, all having different shapes for their respective secondary cleaning portions $12$.

Figure 2 shows the primary cleaning portion $11$ having a first cleaning surface $11a$ and a second cleaning surface $11b$ opposite the first cleaning surface $11a$, and the secondary cleaning portion $12$ having a first cleaning surface $12a$ and a second cleaning surface $12b$ opposite the first cleaning surface $12a$. The first cleaning surface $11a$, $12a$ is disposed on and is a part of the first side of the cleaning article $10$, and the second cleaning surface $11b$, $12b$ is disposed on and is a part of the second side of the cleaning article $10$. The first cleaning surface $11a$ and the second cleaning surface $11b$ of the primary cleaning portion $11$ define a primary cleaning portion thickness $T_p$, which ranges from 2 millimetres to 20 millimetres. For the cleaning article $10$ having its largest plan view dimension ranging from 40 millimetres to 100 millimetres, the most preferred primary cleaning portion thickness $T_p$ is between about 2 millimetres and 6 millimetres. One skilled in the art will appreciate that the thickness $T_p$ is arbitrary and may be chosen with regard to a variety of factors, for example, intended use, overall dimensions, shape, constituent material, dimensions of the secondary cleaning portion.

As is evident from Figures 1 and 2, the secondary cleaning portion $12$ has a predetermined size and shape. The secondary cleaning portion $12$ has a secondary cleaning portion width $W_s$. As used herein, the term "secondary cleaning portion width" means the maximal distance between the secondary cleaning portion edge of the secondary cleaning portion $12$ and the opposite side of the same secondary cleaning portion $12$, measured perpendicular relative to the secondary cleaning portion edge $E_s$ (or if the secondary cleaning portion edge $E_s$ is curved, perpendicular to the tangent of the secondary cleaning portion edge $E_s$). In other words, the secondary cleaning portion width $W_s$ of the secondary cleaning portion $12$ is its greatest inboard dimension measured perpendicular to the secondary cleaning portion edge $E_s$. In a preferred embodiment of the present invention, the secondary cleaning portion width $W_s$ is greater than 1 millimetre. As illustrated in Figure 2, the first cleaning surface $12a$ and the second cleaning surface $12b$ of the secondary cleaning portion $12$ define a secondary cleaning thickness $T_s$, which ranges from 0.01 millimetres to 3 millimetres. In a preferred embodiment of the present invention, the secondary cleaning portion $T_s$ is 1 millimetre. The thickness $T_s$ of the secondary cleaning portion $12$ is less than the thickness $T_p$ of the primary cleaning portion $11$. The respective shapes of the secondary cleaning portion $12$ can be advantageously designed to benefit both the primary cleaning portion $11$ and the secondary cleaning portion $12$. Figure 1 shows a "crescent" or "moon-like" shape for the secondary cleaning portion $12$. This crescent shape serves to maximise the primary cleaning portion $11$ of the cleaning article $10$, and, at the same time, create a secondary cleaning portion $12$ that is particularly suitable for precision cleaning. While the crescent shape for the secondary cleaning portion $12$ is preferred, other shapes are possible. Figures 3a, 3b and 3c show other shapes and these are neither all-inclusive nor exhaustive of the shapes that could be adopted for the secondary cleaning portion $12$.

The primary cleaning portion $11$ and the secondary cleaning portion $12$ have flexural rigidity values $R_p$ and $R_s$, respectively. As used herein, the "flexural rigidity" is proportionate to the force which is necessary to bend, or flex a structure normal to its plane. The flexural rigidity of a particular structure equals the moment of inertia "I" of a cross-sectional portion of this structure multiplied by the modulus of elasticity "E" of this structure (i.e., the flexural rigidity equals R=IE ). The flexural rigidity for simple structures can be theoretically derived as described in elementary textbooks, such as one written by E.P. Popov, "*Mechanics of Materials*", Prentice-Hall Inc., Englewood Cliffs, New Jersey, second edition, 1976.

The secondary cleaning portion **12** has a flexural rigidity value $R_s$ which is greater than the flexural rigidity value $R_p$ for the primary cleaning portion **11**. A commercially available instrument - Dynamic Mechanical Analyser (DMA), Model 7 series/UNIX DMA7, made by Perkin-Elmer Corporation, Mail Station 131, 761 Main Avenue, Norwalk, CT 0689-9966, USA - was used to determine the flexural rigidity value $R_s$ of the secondary cleaning portion **12** of the cleaning article **10** of the present invention. The flexural rigidity $R_s$ of the secondary cleaning portion **12** is typically at least 100 percent greater than the flexural rigidity $R_p$ of the primary cleaning portion **11**. Preferably, the flexural rigidity $R_s$ of the secondary portion **12** is at least 500 percent greater than the flexural rigidity $R_p$ of the primary cleaning portion **11**. More preferably, the flexural rigidity $R_s$ of the secondary cleaning portion **12** is at least about 1000 percent greater than the flexural rigidity $R_p$ of the primary cleaning portion **11**. The Dynamic Mechanical Analyser (DMA) was used to measure the flexural rigidity $R_s$ of the secondary cleaning portion **12**. For the present invention, the flexural rigidity $R_s$ of the secondary cleaning portion **12** was calculated to be about at least 4.5E-7 newtons - metres squared. Before performing the flexural rigidity measurements, the secondary cleaning portion **12** is textured as intended for cleaning by the user.

According to the present invention, the first cleaning surface **12a** and/or the second cleaning surface **12b** of the secondary cleaning portion **12** are preferably textured. Furthermore, the first cleaning surface **11a** and/or the second cleaning surface **11b** of the primary cleaning portion **11** may also be textured. As used herein, the term "textured" refers to discontinuities or non-planar interruptions in what would ordinarily be a generally smooth or planar surface. These discontinuities or non-planar interruptions can comprise projections from or depressions in such a smooth surface. A suitable technique for the present invention is embossing. The embossing may be performed using a pair of embossing rollers, which have embossing mounds arranged according to the respective embossing pattern on their external surfaces. The embossing rollers are arranged and controlled in such a way that the embossed mounds are arranged exactly head-to-head in each case but do not touch each other. The present invention is not limited to such a technique and any technique known in the art such as thermoplastic bonding, hydrogen bonding or air pressurising can be employed to gain the desired embossing pattern and the desired thinness and rigidity for the secondary cleaning portion **12** and if desired the primary cleaning portion **11**.

In a preferred embodiment of the present invention, the cleaning article **10**, which is particularly suitable for contact with the human skin, is directed to facial cleansing. Figure 4 shows that the cleaning article **10** may contain one or more secondary cleaning portions **12**.

Examples of suitable fibre compositions that can be used to make the cleaning article **10** include purely hydrophilic materials or purely hydrophobic materials or mixtures of a purely hydrophilic fibre material and a purely hydrophobic material, which can be present in any desired proportions. Hydrophilic materials comprise, for example, cotton, viscose or flax and hydrophobic materials comprise, for example, polyethylene terephthalate or polypropylene. Preferred embodiments include 100 percent cotton; 100 percent viscose; a minimum of 25 percent viscose and the remainder cotton; and a minimum of 25 percent cotton and the remainder viscose; 50 percent viscose and 50 percent polyethylene terephthalate and 50 percent viscose and 50 percent polypropylene. All these percentages are by weight. The basis weight of the fibres is preferably the same in the primary cleaning portion **11** and the secondary cleaning portion **12**. Nevertheless, the basis weight may be different. As used herein, the term "basis weight" is expressed as the mass of fibres divided by the area measured in the plan view.

The cleaning article **10** can be dry or chemically impregnated in a dry form. Alternatively, the cleaning article **10** can be impregnated with a liquid-cleansing solution. The liquid-cleansing solution can be an aqueous solution or an emulsion in which the continuous phase is aqueous or an oil-based solution in which, for example, the continuous phase is oil. Alternatively, the cleaning article **10** may only contain non-aqueous liquids such as alcohols, ketones and oils.

The present invention also relates to the use of the cleaning article **10** in cleaning around the eye regions. More specifically, the secondary cleaning portion **12** is applied to the regions around the eyes. The thin and rigid nature of the secondary cleaning portion **12** can be utilised most advantageously for precision cleaning around the eyes and the preferable textured feature of the secondary cleaning portion **12** can facilitate the picking up of make-up, dirt, grime and other debris in a superior manner.

## GLOSSARY

| | |
|---|---|
| 10 | Cleaning article |
| 11 | Primary cleaning portion |
| 11a | First cleaning surface of primary cleaning portion |
| 11b | Second cleaning surface of primary cleaning portion |
| 12 | Secondary cleaning portion |
| 12a | First cleaning surface of secondary cleaning portion |

12b Second cleaning surface of secondary cleaning portion
P Perimeter
$R_p$ Flexural rigidity value of primary cleaning portion
$R_s$ Flexural rigidity value of secondary cleaning portion
$T_p$ Thickness of primary cleaning portion
$T_s$ Thickness of secondary cleaning portion
$W_s$ Width of secondary cleaning portion

## Claims

1. Disposable fibrous cleaning article (10) comprising a primary cleaning portion (11) disposed adjacent at least a part of perimeter (P) of said cleaning article (10), said primary cleaning portion (11) having a primary cleaning thickness ($T_p$) ranging from 2 millimetres to 20 millimetres, and at least one secondary cleaning portion (12),
characterised in that

said at least one secondary cleaning portion (12) is disposed adjacent a continuous part of said perimeter (P) of said cleaning article (10), said cleaning portion (12) having a secondary cleaning portion width ($W_s$) greater than 1 millimetre and a secondary cleaning portion thickness ($T_s$), less than said primary cleaning thickness ($T_p$), ranging from 0.01 millimetres to 3 millimetres.

2. Disposable fibrous cleaning article (10) according to claim 1 wherein said at least one secondary cleaning portion (12) has a flexural rigidity value ($R_s$) of about at least 4.5E-7 newtons - metres squared.

3. Disposable fibrous cleaning article (10) according to claim 2 wherein said flexural rigidity value ($R_s$) of the secondary cleaning portion (12) is typically at least 100 percent greater than the flexural rigidity value ($R_p$) of the primary cleaning portion (11).

4. Disposable fibrous cleaning article (10) according to any of the preceding claims wherein said at least one secondary cleaning portion (12) has a first cleaning surface (12a), and a second cleaning surface (12b) opposite said first cleaning surface (12a).

5. Disposable fibrous cleaning article (10) according to claim 4 wherein said first cleaning surface (12a) and/or said second cleaning surface (12b) are textured.

6. Disposable fibrous cleaning article (10) according to any of the preceding claims wherein said cleaning article (10) is suitable for facial cleansing.

7. Disposable fibrous cleaning article (10) according to any of the preceding claims wherein said cleaning article (10) is dry.

8. Disposable fibrous cleaning article (10) according to any of the preceding claims wherein said cleaning article (10) is impregnated with a liquid-cleansing solution.

9. Use of a disposable fibrous cleaning article (10) according to any of the preceding claims wherein said secondary cleaning portion (12) is applied to the regions around the eyes.

Fig. 1

Fig. 2

10

**Fig: 3 a**

12

11

P

10

**Fig: 3 b**

12

P

10

**Fig: 3 c**

12

P

10

**Fig: 4**

P

12

12

European Patent Office

# EUROPEAN SEARCH REPORT

Application Number

EP 96 12 0245

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| A | WO 94 02674 A (PROCTER & GAMBLE) 3 February 1994 <br> * claims 1,8 * | 1-9 | D04H1/54 |
| A | EP 0 245 017 A (MINNESOTA MINING & MFG) 11 November 1987 <br> * the whole document * | 1-9 | |
| A | US 4 948 585 A (SCHLEIN ALLEN P) 14 August 1990 <br> * the whole document * | 1-9 | |

|  | TECHNICAL FIELDS SEARCHED (Int.Cl.6) |
|---|---|
|  | D04H <br> A61K <br> A61F <br> A61L |

The present search report has been drawn up for all claims

| Place of search | Date of completion of the search | Examiner |
|---|---|---|
| THE HAGUE | 2 June 1997 | Barathe, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document